# EUROPEAN PATENT APPLICATION

(11) **EP 1 942 117 A1**
(43) Date of publication of application: **09.07.2008**
(21) Application number: 06425874.2
(22) Date of filing: 29.12.2006
(51) Int. Cl.: C08B 37/08, C08B 11/12, A61K 47/36

(54) **Derivatives of acid polysaccharides**

(71) Applicant: Sigea S.R.L., 34012 Trieste (IT)
(72) Inventor: Stucchi, Luca, 33050 Udine (IT); Bosco, Marco, 34072 Gorizia (IT); Gianni, Rita, 34016 Trieste (IT); Picotti, Fabrizio, 33042 Udine (IT)
(74) Representative: Minoja, Fabrizio

(57) **Abstract**

Acid polysaccharides characterised by the concomitant presence of partial esters with non-polysaccharide carboxylic acids and esters between the acid groups of the initial polysaccharide and the alcohol groups of the repetitive units, with the formation of crosslinking between the polysaccharide chains.

## Description

This invention relates to derivatives of acid polysaccharides esterified to the hydroxyl groups of the polysaccharide with carboxylic acids and crosslinked by means of ester bonds between the free carboxyl groups and hydroxyl groups of the acid polysaccharide.

### Prior art

In view of their chemico-physical and biological-biochemical characteristics, carboxylated polysaccharides, whether natural or semisynthetic, constitute raw materials of considerable interest for a wide variety of applications in the pharmaceutical and cosmetic industries. Some of them, such as hyaluronic acid (HA), are particularly valued for their high level of biotolerability and hydratability, and others, such as carboxymethylcellulose (CMC), etc., for their ability to withstand enzymatic degradation. The choice of material is often connected with cost aspects based on the value and importance of the final application.

Many structural changes have been made to these polysaccharides in recent years to optimise their characteristics and make them as suitable as possible for the desired applications. These changes usually involve the alcohol groups of the repetitive units, the carboxyl groups and, if present, the amino groups.

The prior art as a whole describes two specific structural solutions:
1) simple monoesterification of the alcoholic hydroxyls of the repetitive polysaccharide units, regardless of their nature and origin, with organic acids, without intermolecular crosslinking;
2) formation of autocrosslinking esters between the hydroxyls of the repetitive polysaccharide units and the carboxyls present in the polysaccharide.

EP 0941253 describes the synthesis of HA derivatives with butyric anhydride in a basic environment, to obtain a product which is esterified in terms of hydroxyl functions, not crosslinked, and undergoes no significant modification of its viscoelastic properties in aqueous solution. The process involves the use of the quaternary ammonium salt of HA, and dimethylformamide (DMF) as aprotic solvent.

In EP 341745, starting from HA or from HA wherein the carboxyls are partly esterified with alcohols of various types, including biologically active alcohols, the carboxyl function of HA (or of its ester derivatives, defined as "external") is involved in the formation of intra- or intermolecular esters with the alcoholic hydroxyls of the repetitive units, with consequent crosslinking (defined as "autocrosslinking") and inducement of viscoelastic characteristics which did not exist in the starting polysaccharide. EP 341745 teaches that autocrosslinking is obtained by "activating" the carboxyl by substituting the -OH group with an electron-attractor group X that allows the carbonyl carbon to be attached by a nucleophil (such as the -OH group of the monosaccharide units), with simultaneous detachment of X. The reagents described which are able to activate the carboxyl are the typical, well-known reagents that supply activated esters in peptide synthesis, such as water-soluble carbodiimides, carbonyldiimidazole, carbonyltriazole, N-hydroxysuccinimide, p-nitrophenol, p-nitrophenyltrifluoracetate, and salts of 2-halogen-N-alkylpyridine (especially CMPJ, which is fully exemplified); the reaction is catalysed with triethylamine (TEA). The starting carboxylated polysaccharides (HA or "external" esters) take the form of tetrabutylammonium (TBA) salts soluble in aprotic solvents, such as dimethyl sulphoxide (DMSO), to obtain a single reaction phase. The same patent also claims the use of salts of mono- or bivalent inorganic cations in the same aprotic organic solvents to conduct the same reaction. However, this conflicts with the knowledge of one skilled in the art and with the example given in the patent, as it is well known that these salts are not soluble in solvents such as DMF or DMSO. This is further demonstrated in the same patent, in which only the use of TBA salts in DMSO solvent is exemplified. As the modulation of the chemico-physical, rheological and biological characteristics (in the broadest sense) of products based on carboxylated polysaccharides is crucial for the purpose of the final application(s) required by the market, and the problem does not appear to have been solved to date, at least in the field of this invention, there is considered to be a strongly-felt need for products with modulatable, reproducible, advantageous characteristics, especially their rheological properties in terms of the viscosity of solutions at different concentrations, viscoelasticity and biotolerability.

### Description of the invention

It has now been found, and this finding constitutes the subject of the invention, that the above-mentioned objectives can be achieved with acid polysaccharide derivatives characterised by the concomitant presence of partial esters with non-polysaccharide carboxylic acids and esters between the acid groups of the initial polysaccharide and the alcohol groups of the repetitive units, with the formation of crosslinking between the polysaccharide chains. Analysis of the prior art discussed above indicates the novelty of products which simultaneously present said characteristics of esterification to the hydroxyl groups and crosslinking.

The invention relates to natural or semisynthetic derivatives of acid polysaccharides wherein the alcohol groups of the repetitive units occur, more or less extensively and evenly distributed, in the form of esters with non-polysaccharide carboxylic acids, and the acid groups (uronic or previously introduced into the polymer chain) are esterified, to different extents, with other free alcohol groups present in the polysaccharide chains. This latter type of bond induces crosslinking of the polysaccharide, thus influencing the viscoelastic behaviour of the end products; a suitable choice of acyl residue used to esterify the alcoholic hydroxyls enables other chemico-physical properties, such as hydrophilia/lipophilia and viscosity, to be modulated. The acyl unit being equal, the ratio between the esters to the alcoholic hydroxyls and those involved in crosslinking via carboxyls, which is perfectly modulatable and reproducible, influences the characteristics of the end products (rigid gels, weak gels, products with increased viscosity). The choice of starting acid polysaccharide (natural, such as HA, or semisynthetic, such as CMC) makes it possible to select the biological and biochemical characteristics best suited to the intended final use (such as greater or lower biotolerability, resistance to degradation by enzymes or biological fluids, etc).

The carboxyl groups of the end products can be in acid form or salified with pharmacologically inactive or active inorganic or organic bases.

In view of their chemico-physical and rheological characteristics, the derivatives according to the invention can be used in the pharmaceutical industry as constituents of medical devices of various grades (I, II and III), such as injectable skin fillers, anti-tissue adherence materials, devices for healing sores and wounds, etc., in slow-release galenical formulations, etc.), and in the cosmetics industry (e.g. as constituents with a high moisturising capacity).

The rheological tests conducted demonstrate that the derivatives according to the invention possess rheological properties characterised by viscoelastic behaviour which can be modulated according to the degree of crosslinking of the system, which ranges from that typical of a solution to that characteristic of a strong gel. It was found that the viscosity at low shear rates and the resistance to the force applied were modulatable. Finally, it was found that the polymer mixtures in question have a good ability to recover their viscoelastic properties after a rheological history of imposed stresses.

The invention also relates to the preparation process of these derivatives. It comprises the reaction, in homogenous phase in a protic, polar solvent such as formamide, of the salt of a monovalent inorganic cation, such as sodium or potassium, of the carboxylated polysaccharide selected with an anhydride of an alkyl, aryl, alkyl-aryl or heterocyclic carboxylic acid, such as acetic, butyric, crotonic, benzoic or salicylic anhydride, etc., in the presence of a basic catalyst containing an atom of trisubstituted nitrogen, which may be aliphatic (triethylamine, DBO, DBU, hexamine), aromatic (imidazole, pyridine, DMAP, lutidine, collidine) or heterocyclic, or an inorganic base (K₃PO₄, K₂HPO₄ MₙCO₃, with M=alkaline or alkaline-earth metal). TEA or DMAP is preferably used. A suitable choice of this catalyst directs the reactivity of the anhydride to give more esters with the hydroxyls or promote crosslinking. The interval of the hydroxyl residues involved in ester bonds with the acyl residue deriving from the anhydride can range between 0.001 and 1.0×N, where N is the number of hydroxyls in the repetitive unit. The degree of substitution in terms of the hydroxyl residues involved in the crosslinking is between 0.001 and 0.5 in relation to the repetitive unit.

The starting polysaccharides can be in native form or differently modified according to the chemical functions present.

The usefulness and industrial cost-saving of direct use of an inorganic cation salt is obvious because, as described below, it eliminates the need for preliminary transformation of the polysaccharide into the salt of an organic base (such as TBA) soluble in aprotic solvents such as DMF, DMSO, N-methylpyrrolidone, etc., a lengthy, expensive operation which involves the risk of depolymerisation.

All the tests conducted confirm the perfect modulability and reproducibility of the process according to the invention and the cross-linked derivatives claimed. Using this process, it is possible to plan which rheological aspect should be given priority in order to obtain products that present a mere increase in their viscosity in solution (low degree of crosslinking and different degree of esterification), or derivatives with variable viscoelastic properties ranging from weak gels to strong gels (more cross-linked products).

Acid polysaccharides, either in acid form or in the form of their inorganic salts, consist of regular or irregular repetitive units containing at least one uronic acid residue (glucuronic, galacturonico, guluronic or mannuronic); alternatively, they are polysaccharides derivatised with a residue containing at least one carboxyl function on an alkyl/aryl residue covalently bonded to the polymer with an ether, amino, ester, amide or acetal bond; alternatively, the carboxyl function is introduced by partial oxidative degradation of a saccharide unit in the main chain or on a side branch (e.g. oxidised scleroglucan).

Some specific examples of acid polysaccharides are hyaluronic acid, gellan, xanthan, gum tragacanth, pectins, polygalacturonic acid, alginate, carboxylated derivatives of cellulose such as carboxymethylcellulose, carboxyethylcellulose, carboxymethyl dextran and their derivatives with alkyl/aryl residues. Hyaluronic acid and carboxymethylcellulose are particularly preferred.

Other examples of non-uronic acid polysaccharides useful as starting products for the embodiment according to the invention present residues of malic, maleic, succinic, glutaric, adipic, glutamic and aspartic acids, bonded as esters or amides to the polysaccharide chain.

The carboxyl groups of acid polysaccharides not esterified to the hydroxyl groups could be salified, for example, with alkaline metals, in particular sodium.

The molecular weight of the polysaccharides according to the invention can vary within a wide range, e.g. between 10³ and 10⁷ Daltons.

The products according to the invention can be used as moisturising (dermo-)cosmetic agents, medical aids, intra-articular viscosupplementation agents, anti-tissue adherence filling materials in surgery, and materials for covering wounds or sores.

The products according to the invention can also be advantageously used as a medium for the controlled release or absorption of active constituents, such as anti-inflammatory and antiproliferative drugs.

The following examples illustrate the invention in greater detail.

### EXAMPLES

The ¹H NMR analyses are conducted in D₂O with a Bruker Avance 400 spectrometer equipped with a 5 mm multinuclear probe with gradient z, at 300°K. The analyses also use diffusion-ordered experiments (DOSY: Diffusion Ordered Spectroscopy).

The rheological tests were performed with a Rheostress Haake RS 150 controlled-stress rotational rheometer.

The determination of the percentage of ester groups of the polysaccharide alcoholic hydroxyls with the various non-polysaccharide carboxylic acids (DE) and with the polysaccharide carboxyls (AUC) was expressed as the molar ratio between the moles of ester and polysaccharide by means of ¹H NMR spectroscopic analysis.

A sample of esterified, cross-linked polysaccharide swollen in formamide containing excess propylamine is left under magnetic agitation at ambient temperature for 16 hours. The polysaccharide is recovered by precipitation in acetone, washed with acetone and dried. The solid is analysed by ¹H NMR. The acylation reaction of the amine with carboxyl esters (Michael B. Smith and Jerry March - March's Advanced Organic Chemistry, 5th ed., Wiley Interscience, page 510) is exploited to determine the esters previously used in the crosslinking; this methodology is selective, because the bland conditions used only involve unstable esters. The esters to the hydroxyls are analysed, again with ¹H NMR, exploiting the different resonance of the methyls and methylenes of the acyl residues bonded to the hydroxyls compared with the other polymer signals.

### Example 1: Synthesis of cross-linked hyaluronic acid acetate sodium salt, degree of autocrosslinking (AUC): 0.07; degree of esterification (DE): 0.19

1.00 g of hyaluronic acid in the form of sodium salt, with a molecular weight of approx. 300 kD, equal to 2.49 mmols of monomer units, was dissolved in 33 mL of formamide at 95°C, under nitrogen flow, with mechanical agitation. The solution was then cooled to ambient temperature, and 352 µL of acetic anhydride (3.73 mmols) and 312 µL of triethylamine (2.24 mmols) were added. After 16 hours' reaction, a further 521 µL of triethylamine (3.74 mmols) was added, and the system was left to agitate for another 6 hours.

The gel was then transferred, slowly and under constant agitation, into 100 mL of an 0.2 M solution of NaCl, transferred to a dialysis membrane (cut-off 12,000 D) and dialysed, firstly against 0.2 M NaCl and secondly against demineralised water. Finally, it was frozen and freeze-dried.

0.94 g of white lyophilisate was obtained.

The product, analysed in accordance with the method described above, presented AUC: 0.07; DE: 0.19.

### Example 2: Synthesis of cross-linked hyaluronic acid acetate sodium salt, degree of autocrosslinking (AUC): 0.05; degree of esterification (DE): 0.16

1.00 g of hyaluronic acid in the form of sodium salt, with a molecular weight of approx. 300 kD, equal to 2.49 mmols of monomer units, was dissolved in 33 mL of formamide at 80°C, under nitrogen flow, with mechanical agitation. The solution was then cooled to ambient temperature, and 235 µL of acetic anhydride (2.49 mmols) and 311 µL of triethylamine (2.23 mmols) were added. After 16 hours' reaction, a further 320 µL of triethylamine (2.29 mmols) was added, and the system was left to agitate for another 6 hours.

The gel was then transferred, slowly and under constant agitation, into 70 mL of an 0.2 M solution of NaCl, and neutralised with KH₂PO₄. After approx. 16 hours' agitation, the system was transferred to a dialysis membrane (cut-off 12000 D) and dialysed, firstly against 0.2 M NaCl and secondly against demineralised water. Finally, it was frozen and freeze-dried.

0.9 g of white lyophilisate was obtained.

The product, analysed in accordance with the method described above, presented AUC: 0.05; DE: 0.16.

### Example 3: Synthesis of cross-linked hyaluronic acid acetate sodium salt, degree of autocrosslinking (AUC): 0.05; degree of esterification (DE): 0.15

1.00 g of hyaluronic acid in the form of sodium salt, with a molecular weight of approx. 300 kD, equal to 2.49 mmols of monomer units, was dissolved in 33 mL of formamide at 95°C, under nitrogen flow, with mechanical agitation. The solution was then cooled to ambient temperature, and 235 µL of acetic anhydride (2.49 mmols) and 277 µL of triethylamine (1.99 mmols) were added. After 16 hours' reaction, a further 417 µL of triethylamine (3.00 mmols) was added, and the system was left to agitate for another 6 hours.

The gel was then transferred, slowly and under constant agitation, into 70 mL of an 0.2 M solution of NaCl, and then to a dialysis membrane (cut-off 12000 D) and dialysed, firstly against 0.2 M NaCl and secondly against demineralised water. Finally, it was frozen and freeze-dried.

0.98 g of white lyophilisate was obtained.

The product, analysed in accordance with the method described above, presented AUC: 0.05; DE: 0.15.

### Example 4: Synthesis of cross-linked hyaluronic acid acetate sodium salt, degree of autocrosslinking (AUC): 0.03; degree of esterification (DE): 0.12

1.02 g of hyaluronic acid in the form of sodium salt, with a molecular weight of approx. 300 kD, equal to 2.54 mmols of monomer units, was dissolved in 33 mL of formamide at 95°C, under nitrogen flow, with mechanical agitation. The solution was then cooled to ambient temperature, and 120 µL of acetic anhydride (1.27 mmols) and 140 µL of triethylamine (1.01 mmols) were added. After 22 hours a further 140 µL of triethylamine (1.01 mmols) was added, and the gelatinous mass obtained was left to agitate for another 6 hours.

The reaction mixture was then transferred into 100 mL of an 0.2 M solution of NaCl, poured into a dialysis membrane (cut-off 12,000 D) and dialysed, firstly against an 0.2 M solution of NaCl and secondly against demineralised water. Finally, the ample was frozen and freeze-dried, and 1.00 g of white lyophilisate was obtained.

The product, analysed in accordance with the method described above, presented AUC: 0.03; DE: 0.12.

### Example 5: Synthesis of cross-linked hyaluronic acid acetate sodium salt, degree of autocrosslinking (AUC): 0.01; degree of esterification (DE): 0.05

1.00 g of hyaluronic acid in the form of sodium salt, with a molecular weight of approx. 300 kD, equal to 2.49 mmols of monomer units, was dissolved in 33 mL of formamide at 95°C, under nitrogen flow, with mechanical agitation. The solution was then cooled to ambient temperature, and 82 µL of acetic anhydride (0.87 mmols) and 87 µL of triethylamine (0.63 mmols) were added. After 19 hours a further 348 µL of triethylamine (2.52 mmols) was added, and the gelatinous mass obtained was left to agitate for another 6 hours.

The reaction mixture was then transferred into 100 mL of demineralised water, poured into a dialysis membrane (cut-off 12000 D) and dialysed, firstly against an 0.2 M solution of NaCl and secondly against demineralised water. Finally, the sample was frozen and freeze-dried, and 0.89 g of white lyophilisate was obtained.

The product, analysed in accordance with the method described above, presented AUC: 0.01; DE: 0.05.

### Example 6: Synthesis of cross-linked hyaluronic acid acetate sodium salt, degree of autocrosslinking (AUC): 0.03; degree of esterification (DE): 0.08

2.00 g of hyaluronic acid in the form of sodium salt, with a molecular weight of approx. 300 kD, equal to 4.99 mmols of monomer units, was solubilised in 67 mL of formamide at 80°C, under nitrogen flow, with mechanical agitation. The solution was then cooled to ambient temperature, and 710 µL of acetic anhydride (7.52 mmols) and 970 µL of triethylamine (6.97 mmols) were added.

After 2 hours 30 minutes the gel was transferred into 350 mL of an 0.2 M solution of NaCl, and the mixture was transferred to a dialysis membrane (cut-off 12000 D) and dialysed, firstly against 0.2 M NaCl and then exhaustively against demineralised water. Finally, the sample was frozen and freeze-dried, and 2.10 g of white lyophilisate was obtained.

The product, analysed in accordance with the method described above, presented AUC: 0.03; DE: 0.08.

### Example 7: Synthesis of cross-linked hyaluronic acid acetate sodium salt, degree of autocrosslinking (AUC): 0.03; degree of esterification (DE): 0.23

1.01 g of hyaluronic acid in the form of sodium salt, with a molecular weight of approx. 300 kD, equal to 2.49 mmols of monomer units, was dissolved in 33 mL of formamide at 95°C, under nitrogen flow, with mechanical agitation. The solution was then cooled to ambient temperature, and 240 µL of acetic anhydride (2.54 mmols) and a solution of 0.28 g of dimethylaminopyridine (2.29 mmols), dissolved in 2 mL of formamide, were added. After 19 hours' reaction, a further 0.56 µL of dimethylaminopyridine (4.58 mmols), dissolved in 4 mL of formamide, was added, and the system was left to agitate for another 5 hours.

The gel was then transferred, slowly and under constant agitation, into 150 mL of an 0.2 M solution of NaCl, and neutralised with KH₂PO₄. It was then transferred to a dialysis membrane (cut-off 12000 D) and dialysed, firstly against 0.2 M NaCl and secondly against demineralised water. Finally, it was frozen and freeze-dried.

1.09 g of white lyophilisate was obtained.

The product, analysed in accordance with the method described above, presented AUC: 0.03; DE: 0.23.

### Example 8: Synthesis of cross-linked hyaluronic acid propionate sodium salt, degree of autocrosslinking (AUC): 0.05; degree of esterification (DE): 0.06

1.02 g of hyaluronic acid in the form of sodium salt, with a molecular weight of approx. 300 kD, equal to 2.54 mmols of monomer units, was dissolved in 33 mL of formamide at 95°C, under nitrogen flow, with mechanical agitation. The solution was then cooled to ambient temperature, and 330 µL of propionic anhydride (2.57 mmols) and 320 µL of triethylamine (2.30 mmols) were added.

The reaction mixture was then maintained under agitation for approximately 3 hours, and a further 320 µL of triethylamine (2.30 mmols) was added. After 2 hours, the gel was transferred into 100 mL of demineralised water and neutralised with KH₂PO₄. It was then transferred to a dialysis membrane (cut-off 12000 D) and dialysed, firstly against 0.2 M NaCl and secondly against demineralised water.

Finally, the sample was frozen and freeze-dried, and 0.96 g of white lyophilisate was obtained.

The product, analysed in accordance with the method described above, presented AUC: 0.05; DE: 0.06.

### Example 9: Synthesis of cross-linked hyaluronic acid butyrate sodium salt, degree of autocrosslinking (AUC): 0.06; degree of esterification (DE): 0.18

1.00 g of hyaluronic acid in the form of sodium salt, with a molecular weight of approx. 300 kD, equal to 2.52 mmols of monomer units, was dissolved in 33 mL of formamide at 95°C, under nitrogen flow, with mechanical agitation. The solution was then cooled to ambient temperature, and 610 µL of butyric anhydride (3.73 mmols) and 310 µL of triethylamine (2.23 mmols) were added.

The reaction mixture was then maintained under agitation for approximately 20 hours, and a further 550 µL of triethylamine (3.95 mmols) was added. After 6 hours, the gel was transferred into 150 mL of ultrapure water, transferred to a dialysis membrane (cut-off 12000 D) and dialysed, firstly against 0.2 M NaCl and secondly against ultrapure water.

Finally, the sample was frozen and freeze-dried, and 0.95 g of white lyophilisate was obtained.

The product, analysed in accordance with the method described above, presented AUC: 0.06; DE: 0.18.

### Example 10: Synthesis of cross-linked hyaluronic acid butyrate sodium salt, degree of autocrosslinking (AUC): 0.05; degree of esterification (DE): 0.15

1.01 g of hyaluronic acid in the form of sodium salt, with a molecular weight of approx. 300 kD, equal to 2.52 mmols of monomer units, was dissolved in 33 mL of formamide at 95°C, under nitrogen flow, with mechanical agitation. The solution was then cooled to ambient temperature, and 490 µL of butyric anhydride (3.00 mmols) and 280 µL of triethylamine (2.01 mmols) were added.

The reaction mixture was then maintained under agitation for approximately 5 hours, and a further 280 µL of triethylamine (2.01 mmols) was added. After 16 hours, the gel was transferred into 150 mL of ultrapure water, transferred to a dialysis membrane (cut-off 12000 D) and dialysed, firstly against 0.2 M NaCl and secondly against ultrapure water.

Finally, the sample was frozen and freeze-dried, and 1.00 g of white lyophilisate was obtained.

The product, analysed in accordance with the method described above, presented AUC: 0.05; DE: 0.15.

### Example 11: Synthesis of cross-linked hyaluronic acid butyrate sodium salt, degree of autocrosslinking (AUC): 0.03; degree of esterification (DE): 0.08

1.00 g of hyaluronic acid in the form of sodium salt, with a molecular weight of approx. 300 kD, equal to 2.49 mmols of monomer units, was dissolved in 33 mL of formamide at 80°C, under nitrogen flow, with mechanical agitation. The solution was then cooled to ambient temperature, and 360 µL of butyric anhydride (2.20 mmols) and 311 µL of triethylamine (2.23 mmols) were added.

The reaction mixture was then maintained under agitation for approximately 16 hours, and a further 311 µL of triethylamine (2.23 mmols) was added. After 6 hours 30 minutes the gel was transferred into approx. 40 mL of an 0.2 M solution of NaCl, transferred to a dialysis membrane (cut-off 12000 D), and exhaustively dialysed against demineralised water. Finally, the sample was frozen and freeze-dried, and 0.90 g of white lyophilisate was obtained.

The product, analysed in accordance with the method described above, presented AUC: 0.03; DE: 0.08.

### Example 12: Synthesis of cross-linked hyaluronic acid butyrate sodium salt, degree of autocrosslinking (AUC): 0.03; degree of esterification (DE): 0.1

1.00 g of hyaluronic acid in the form of sodium salt, with a molecular weight of approx. 300 kD, equal to 2.52 mmols of monomer units, was dissolved in 33 mL of formamide at 95°C, under nitrogen flow, with mechanical agitation. The solution was then cooled to ambient temperature, and 244 µL of butyric anhydride (1.49 mmols) and 173 µL of triethylamine (1.24 mmols) were added.

The reaction mixture was then maintained under agitation for approximately 16 hours, and a further 208 µL of triethylamine (1.49 mmols) was added. After 6 hours, the gel was transferred into 100 mL of ultrapure water, transferred to a dialysis membrane (cut-off 12000 D) and dialysed, firstly against 0.2 M NaCl and secondly against ultrapure water.

Finally, the sample was frozen and freeze-dried, and 0.95 g of white lyophilisate was obtained.

The product, analysed in accordance with the method described above, presented AUC: 0.03; DE: 0.1.

### Example 13: Synthesis of cross-linked hyaluronic acid butyrate sodium salt, degree of autocrosslinking (AUC): 0.02; degree of esterification (DE): 0.06

1.00 g of hyaluronic acid in the form of sodium salt, with a molecular weight of approx. 300 kD, equal to 2.49 mmols of monomer units, was dissolved in 33 mL of formamide at 95°C, under nitrogen flow, with mechanical agitation. The solution was then cooled to ambient temperature, and 210 µL of butyric anhydride (1.28 mmols) and 140 µL of triethylamine (1.0 mmols) were added.

The reaction mixture was then maintained under agitation for 17 hours, and a further 320 µL of triethylamine (3.00 mmols) was added. After 6 hours 30 minutes the gel was transferred into 150 mL of ultrapure water, transferred to a dialysis membrane (cut-off 12000 D) and dialysed, firstly against 0.2 M NaCl and secondly against ultrapure water.

Finally, the sample was frozen and freeze-dried, and 0.99 g of white lyophilisate was obtained.

The product, analysed in accordance with the method described above, presented AUC: 0.02; DE: 0.06.

### Example 14: Synthesis of cross-linked hyaluronic acid butyrate sodium salt, degree of autocrosslinking (AUC): 0.01; degree of esterification (DE): 0.06

1.01 g of hyaluronic acid in the form of sodium salt, with a molecular weight of approx. 300 kD, equal to 2.52 mmols of monomer units, was dissolved in 33 mL of formamide at 95°C, under nitrogen flow, with mechanical agitation. The solution was then cooled to ambient temperature, and 125 µL of butyric anhydride (0.76 mmols) and 75 µL of triethylamine (0.5 mmols) were added.

The reaction mixture was then maintained under agitation for 18 hours, and a further 740 µL of triethylamine (5.32 mmols) was added. After 6 hours, the system was transferred into 200 mL of demineralised water, neutralised with KH₂PO₄, transferred to a dialysis membrane (cut-off 12000 D) and dialysed, firstly against 0.2 M NaCl and secondly against water.

Finally, the sample was frozen and freeze-dried, and 0.82 g of white lyophilisate was obtained.

The product, analysed in accordance with the method described above, presented AUC: 0.01; DE: 0.06.

### Example 15: Synthesis of cross-linked hyaluronic acid butyrate sodium salt, degree of autocrosslinking (AUC): 0.03; degree of esterification (DE): 0.55

0.50 g of hyaluronic acid butyrate in the form of sodium salt (degree of butyration, namely moles of butyric groups on moles of polysaccharide, equal to approx. 0.54, MW=439 g/mol, 1.14 mmols) was dissolved in 17 mL of formamide at 60°C, under nitrogen flow and with mechanical agitation. The solution was then cooled to ambient temperature, and 186 µL of butyric anhydride (1.14 mmols) and 143 µL of triethylamine (1.03 mmols) were added.

The reaction mixture was then maintained under agitation for 19 hours, and a further 143 µL of triethylamine (1.03 mmols) was added. After 4½ hours the system was transferred into 100 mL of an 0.2 M solution of NaCl, and neutralised with KH₂PO₄- It was then transferred to a dialysis membrane (cut-off 12000 D) and dialysed, firstly against 0.2 M NaCl and secondly against demineralised water.

Finally, the sample was frozen and freeze-dried, and 0.45 g of white lyophilisate was obtained.

The product, analysed in accordance with the method described above, presented AUC: 0.03; DE: 0.55.

### Example 16: Synthesis of cross-linked hyaluronic acid crotonate sodium salt, degree of autocrosslinking (AUC): 0.05; degree of esterification (DE): 0.08

1.02 g of hyaluronic acid in the form of sodium salt, with a molecular weight of approx. 300 kD, equal to 2.54 mmols of monomer units, was dissolved in 32 mL of formamide at 95°C, under nitrogen flow, with mechanical agitation. The solution was then cooled to ambient temperature, and 380 µL of crotonic anhydride (2.56 mmols) and 320 µL of triethylamine (2.30 mmols) were added.

The reaction mixture was maintained under agitation for 18 hours, and a further 320 µL of triethylamine (2.30 mmols) was added. After 7 hours 30 minutes the gel was transferred into 150 mL of ultrapure water, transferred to a dialysis membrane (cut-off 12000 D) and dialysed, firstly against an aqueous solution of 0.2 M NaCl and secondly against ultrapure water.

Finally, the sample was frozen and recovered by freeze-drying.

0.45 g of white lyophilisate was obtained.

The product, analysed in accordance with the method described above, presented AUC: 0.05; DE: 0.08.

### Example 17: Synthesis of cross-linked hyaluronic acid isovalerate (or 3-methyl-butyrate) sodium salt, degree of autocrosslinking (AUC): 0.03; degree of esterification (DE): 0.08

0.48 g of hyaluronic acid in the form of sodium salt, with a molecular weight of approx. 300 kD, equal to 1.21 mmols of monomer units, was dissolved in 16 mL of formamide at 95°C, under nitrogen flow, with mechanical agitation. The solution was then cooled to ambient temperature, and 240 µL of isovaleric anhydride (1.20 mmols) and 150 µL of triethylamine (1.08 mmols) were added.

The reaction mixture was maintained under agitation for 16 hours, and a further 167 µL of triethylamine (1.20 mmols) was added. After 6 hours, the gel was transferred into 100 mL of demineralised water and neutralised with KH₂PO₄, and 2.5 g of NaCl was added. It was then transferred to a dialysis membrane (cut-off 12000 D) and dialysed against water.

Finally, the sample was frozen and recovered by freeze-drying.

0.89 g of white lyophilisate was obtained.

The product, analysed in accordance with the method described above, presented AUC: 0.03; DE: 0.08.

### Example 18: Synthesis of cross-linked hyaluronic acid benzoate sodium salt, degree of autocrosslinking (AUC): 0.03; degree of esterification (DE): 0.07

1.00 g of hyaluronic acid in the form of sodium salt, with a molecular weight of approx. 300 kD, equal to 2.49 mmols of monomer units, was dissolved in 33 mL of formamide at 80°C, under nitrogen flow, with mechanical agitation. The solution was then cooled to ambient temperature, and 564 mg of benzoic anhydride (2.49 mmols) and 312 µL of triethylamine (2.24 mmols) were added.

The reaction mixture was then maintained under agitation for 16 hours, and a further 346 µL of triethylamine (2.49 mmols) was added. After 5 hours 30 minutes the gel was transferred into 100 mL of an 0.2 M solution of NaCl, transferred to a dialysis membrane (cut-off 12000 D), and exhaustively dialysed against demineralised water.

Finally, the sample was frozen and recovered by freeze-drying.

1.0 g of white lyophilisate was obtained.

The product, analysed in accordance with the method described above, presented AUC: 0.03; DE: 0.07.

### Example 19: Reaction of hyaluronic acid with O-acetylsalicylic anhydride and cross-linked sodium salt, degree of autocrosslinking (AUC): 0.03; degree of esterification (DE): 0.02

0.52 g of hyaluronic acid in the form of sodium salt, with a molecular weight of approx. 300 kD, equal to 1.29 mmols of monomer units, was dissolved in 17 mL of formamide at 95°C, under nitrogen flow, with mechanical agitation. The solution thus obtained was cooled to ambient temperature, and a solution of 0.4170 g of O-acetylsalicylic anhydride in 5 mL of formamide (1.22 mmols) and 152 µL of triethylamine (1.09 mmols) was added. The reaction mixture was then mantained under agitation for 16 hours and a furter 202 µL of triethylamine were added (1.45 mmols). After 5 hours 30 minutes the gel was transferred into 100 mL of an 0.2 M aqueous solution of NaCl and dialysed against demineralised water (cut-off 12000 D).

The product, analysed in accordance with the method described above, presented AUC: 0.03; DE: 0.02.

### Example 20: Synthesis of cross-linked carboxymethylcellulose acetate sodium salt, degree of autocrosslinking (AUC): 0.01; degree of esterification (DE): 0.05

1.00 g of carboxymethylcellulose sodium salt (degree of substitution, namely moles of carboxyl groups on moles of polysaccharide, equal to approx. 0.8, MW=224.4 g/mol, 4.46 mmols), was dissolved in 52 mL of formamide at 80°C, under nitrogen flow, with mechanical agitation. The solution was then cooled to ambient temperature, and 420 µL of acetic anhydride (4.44 mmols) and 560 µL of triethylamine (4.02 mmols) were added.

After 18 hours, a further 560 µL of triethylamine (4.02 mmols) was added to the solution, and the gel thus obtained was kept under agitation for another 6 hours. The gel was then transferred into approx. 200 mL of demineralised water and neutralised with a 1 N solution of HCl. 1.7 g of NaCL was added and the mixture was dialysed, firstly against 0.2 M NaCl and secondly against water. Finally, the sample was frozen and recovered by freeze-drying.

0.98 g of white lyophilisate was obtained.

The product, analysed in accordance with the method described above, presented AUC: 0.01; DE: 0.05.

### RHEOLOGICAL CHARACTERISATION OF ESTERIFIED AND CROSS-LINKED DERIVATIVES

The tests were conducted on samples swollen in saline at the concentration of 1% w/w.

The results of the rheological characterisation of some samples representative of the products obtained are set out in Table 1.

| **Example no.** | **shear viscosity *η*₀ [Pa.s]** | **shear viscosity *η* at shear rate 1000s-1 [Pa.s]** | **storage modulus G' at 1Hz [Pa]** | **loss modulus G" at 1Hz [pa]** | **D.S. ester** | **D.S. Links** |
|---|---|---|---|---|---|---|
| linear native | 0,1 | 0,05 | np | np | 0 | 0 |
| linear butyrate ester | 0,008 | 0,007 | np | np | 0,07 | 0 |
| 4 | 10.000,0 | 0,09 | 60 | 21 | 0,12 | 0,03 |
| 5 | 0,9 | 0,05 | 0,8 | 1,8 | 0,05 | 0,01 |
| 6 | 8.000,0 | 0,2 | 32 | 12 | 0,08 | 0,03 |
| 10 | 100.000,0 | 0,07 | 180 | 40 | 0,15 | 0,05 |
| 13 | 30,0 | 0,09 | 2,9 | 3,3 | 0,06 | 0,02 |
| 14 | 0,6 | 0,028 | np | np | 0,06 | 0,01 |
| 15 | 0,07 | 11000 | 45 | 16 | 0,55 | 0,03 |
| 18 | 10.200,0 | 0,08 | 50 | 18 | 0,07 | 0,03 |

## Claims

1. Acid polysaccharides **characterised by** the concomitant presence of partial esters with non-polysaccharide carboxylic acids and esters between the acid groups of the initial polysaccharide and the alcohol groups of the repetitive units, with the formation of crosslinking between the polysaccharide chains.

2. Acid polysaccharides as claimed in claim 1, comprising repetitive units containing at least one uronic acid residue.

3. Acid polysaccharides as claimed in claim 1, consisting of polysaccharides derivatised with a residue containing at least one carboxyl function on an alkyl/aryl residue covalently bonded to the polymer with an ether, amino, ester, amide or acetal bond.

4. Acid polysaccharides as claimed in claim 1, wherein the carboxyl function is introduced by partial oxidative degradation of a saccharide unit in the main chain or on a side branch (oxidised scleroglucan).

5. Acid polysaccharides as claimed in claim 1, wherein the starting polysaccharide is selected from among hyaluronic acid, gellan, xanthan, gum tragacanth, pectins, polygalacturonic acid and alginate.

6. Acid polysaccharides as claimed in claim 1, wherein the starting polysaccharide is selected from among carboxymethylcellulose, carboxyethylcellulose, carboxymethyldextran and their derivatives with alkyl/aryl residues.

7. Acid polysaccharides as claimed in claim 1, wherein the starting polysaccharide is selected from among hyaluronic acid and carboxymethylcellulose.

8. Acid polysaccharides as claimed in any of claims 1 to 7, wherein the non-polysaccharide carboxylic acids are chosen from among malic, maleic, succinic, glutaric, adipic, glutamic, and aspartic acid.

9. Acid polysaccharides as claimed in any of claims 1 to 8, wherein the carboxyl functions are present in acid or salified form.

10. Acid polysaccharides as claimed in claim 9, wherein the carboxyl functions are salified with alkaline metals, in particular Na⁺.

11. Acid polysaccharides as claimed in any of claims 1 to 10, **characterised by** a molecular weight of between 10³ and 10⁷ Daltons.

12. Acid polysaccharides as claimed in any of claims 1 to 10, **characterised by** a molecular weight of between 10⁴ and 5x10⁵ Daltons.

13. Acid polysaccharides as claimed in any of claims 1 to 11, wherein the esters on the hydroxyls present a degree of substitution ranging between 0.001 and 1.0xN, where N is the number of hydroxyls present in the repetitive unit.

14. Acid polysaccharides as claimed in claim 12, wherein the esters on the hydroxyls present a degree of substitution ranging between 0.001 and 0.5 in relation to the repetitive unit.

15. Process for the preparation of the acid polysaccharides claimed in claims 1-14, which comprises solubilisation of the carboxylated polysaccharide in formamide and the addition of an anhydride of the non-polysaccharide acid and a base.

16. Process as claimed in claim 15, wherein the anhydride is the anhydride of a linear- or branched-chain aryl / alkyl carboxylic acid.

17. Process as claimed in claim 16, wherein the anhydride is chosen from the group of acetic, propionic, butyric, citric, benzoic, isovaleric and crotonic anhydride.

18. Process as claimed in any of claims 15 to 17, wherein the base is an organic base containing an atom of trisubstituted nitrogen, which may be aliphatic, aromatic or heterocyclic, or an inorganic base.

19. Process as claimed in claim 18, wherein the base is triethylamine.

20. Acid polysaccharides as claimed in claims 1-14 for use as moisturising agents, medical aids, intra-articular viscosupplementation agents, anti-tissue adherence filling materials in surgery, and materials for covering wounds and sores.

21. Acid polysaccharides as claimed in claims 1-14 as a medium for the controlled release or absorption of anti-inflammatory and antiproliferative active constituents.
